# EUROPEAN PATENT APPLICATION

(11) **EP 1 134 287 A1**
(43) Date of publication of application: **19.09.2001**
(21) Application number: 00810196.6
(22) Date of filing: 08.03.2000
(51) Int. Cl.: C12N 15/63, C12N 15/67, C12N 15/85

(54) **A system to control the expression of a given gene using another gene that encodes a polypeptide with recombinant activity**

(71) Applicant: UNIVERSITE DE GENEVE, CH-1211 Geneve 4 (CH)
(72) Inventor: Herrera, Pedro L., 1241 Puplinges (CH); Fuhrmann-Benzakein, Edya, 1206 Geveva (CH); Vassali, Jean-Dominique, 1246 Corsier (CH); METZGER, Daniel, F-67404 ILLKIRCH Cedex (FR); CHAMBON, Pierre, F-67404 ILLKIRCH Cedex (FR)
(74) Representative: Moinas, Michel

(57) **Abstract**

The invention, in the field of medecine and molecular biology, concerns a system to control the expression of a gene of interest, either in vitro or in vivo. It involves a first DNA sequence comprising a gene of interest linked in functional relation to a promoter, and a second DNA sequence comprising a second gene that encodes a polypeptide having a recombinant activity specific for target DNA sequences, and two of said target DNA sequences flanking one of the said two DNA sequences. According to the invention, the said second DNA sequence is located between the said promoter and said gene of interest. Another object of the invention is a DNA vector for the transfection of cells characterized in that it contains at least the system of the invention. The invention also concerns a self excision DNA cassette constituted by a DNA sequence flanked by target DNA sequences comprising at least a gene that encodes an inducible polypeptide having a recombinant activity specific for said target DNA sequences. This self-excision DNA cassette may be used as a blocking sequence that prevent the expression of a given gene under the control of a promoter.

## Description

The invention, in the field of medecine and molecular biology, concerns a system to control the expression of a gene of interest, either in vitro or in vivo. It involves a first DNA sequence comprising a gene of interest linked in functional relation to a promoter, and a second DNA sequence comprising a second gene that encodes a polypeptide having a recombinant activity specific for target DNA sequences, and two of said target DNA sequences flanking, directly or not, one of the said two DNA sequences. According to the invention, the said second DNA sequence is located between the said promoter and said gene of interest. Another object of the invention is a DNA vector for the transfection of cells characterized in that it contains at least the system of the invention. The invention also concerns a self excision DNA cassette constituted by a DNA sequence flanked by target DNA sequences comprising at least a gene that encodes an inducible polypeptide having a recombinant activity specific for said target DNA sequences. This self-excision DNA cassette may be used as a blocking sequence that prevents the expression of a given gene under the control of a promoter.

### Background of the invention

The emergence of new molecular tools has allowed the design and generation of transgenic mice carrying subtle mutations, whose expression can be targeted both spatially and temporally, by using cell type-specific or inducible promoters.

A number of different site specific recombinase systems has been identified as well as their target DNA sequences, including the Cre/lox system of bacteriophage P1, the FLP/FRT system of yeast, the Gin recombinase of phage Mu, the Pin recombinase of E.Coli, and the R/RS system of pSR1 plasmid. The site-specific recombinase of those systems may also be modified to bear new features. For instance, Cre-ER^{T}, in which the ligand-binding domain of a mutated estrogen receptor (ER^{T}) that recognizes the anti-estrogen tamoxifen or its derivative 4-hydroxytamoxifen (4-OHT), has been added to Cre (Feil P. et al., *Biochem Biophys Res Commun* 237, 752-7, 1997). In the absence of 4-OHT, Cre-ER^{T} remains in the cytoplasm, while administration of 4OHT results in its migration into the nucleus, as expected for a steroid hormon receptor-ligand complex. Accordingly, Cre-mediated recombination of genomic DNA is 4-OHT-dependent in mice bearing a Cre-ER^{T} transgene.

The site-specific recombinase system was initially devised to *inactivate* genes in selected tissues, rather than in the whole organism. A subsequent development of this system has allowed, *in vivo,* the exquisite temporal control of the recombinant activity of Cre in selected cells (Sauer et al., Methods Enzymol 225, 890-900, 1993). In general a site-specific recombinase system consists of three elements : two pairs of DNA sequence (the site-specific recombination) and a specific enzyme (the site-specific recombinase). This sytem has been used in to control expression of a gene of interest in a vector or a DNA construct to make genetically modified plants or animals, for instance. US 5.977.441 describes a possible utilisation of this system wherein a cell is transfected with a series of DNA sequences comprising a first gene whose expression results in an altered plant phenotype linked to a transiently active promoter, the gene and promoter being separated by a blocking sequence flanked on either side by specific excision sequences, a second gene in another DNA transgene that encodes a recombinase specific for the specific excision sequences linked to a repressible promoter, and a third gene in another DNA sequence that encodes the repressor specific for the repressible promoter. Thus, experimental or therapeutic designs involving the conditional expression of genes often require the use of two different transgenes, the so-called binary systems.

Thomas, K.R. et al *(genes & development,* 13 : 1524-1528, 1999) described a procedure that directs the self-induced deletion of DNA sequences as they pass through the male germ line of mice. A cell specific promoter was used to drive expression of the Cre-recombinase gene. A sequence encoding Cre was placed upstream of the sequence comprising the selectable marker Neo^{r} under the control of a constitutive promoter, and the two sequences flanked with loxP elements. This system allows the elimination of an undesirable gene in the male germ line.

At present, no monogenic system allows controlling the expression of a given gene, i.e. either its activation or inactivation. For such purpose, both the inductive and effector elements must be placed together within the same molecule of DNA. Such a procedure simplifies most experimental designs involving conditional gene expression. In addition, it functions independently of the proliferative state of target cells.

### Summary of the invention

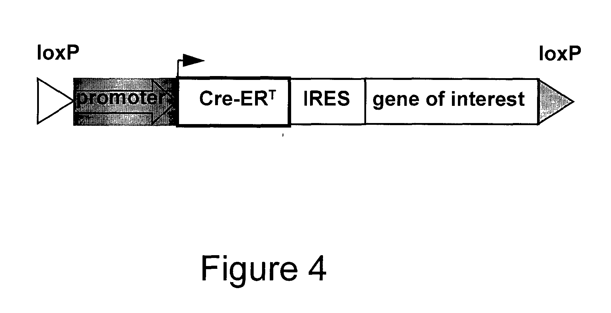

The invention concerns a system to control the expression of a gene of interest comprising a first DNA sequence containing the gene of interest linked in functional relation to a promoter, and a second DNA sequence comprising a second gene that encodes a polypeptide (recombinase) having a recombinant activity specific for target DNA sequences, and two of said target DNA sequences flanking one of the said two DNA sequences. According to the invention, the said second DNA sequence, i.e. the sequence comprising the recombinase gene, is located between the said promoter of the gene of interest and said gene of interest. In a preferred embodiment, the said polypeptide having a recombinant activity is inducible. Preferably, said second gene, i.e. the recombinase gene, is located such that at least said second gene is translated from a single RNA transcript which comprises both the messenger RNA transcribed from the gene of interest and the messenger RNA transcribed from the said second gene. In another embodiment, as represented in figure 4, an internal ribosome entry site (ires sequence) may be placed upstream of the gene of interest. In this last embodiment, both the gene of interest and the second gene may be translated from this said single RNA transcript. When the polypeptide having the recombinant activity is induced, the DNA sequence flanked by the target DNA sequence is excised thus modifying the structure of the system and, consequently, the expression of the gene of interest.

Here we show that recombinases can be used to achieve the temporal control of expression of a gene of interest without the necessity of developing a binary system, i.e. without a requirement for two different transgenes. According to the invention, the recombinase-encoding sequence, the Cre-ER^{T} gene for instance, flanked by two of its target DNA sequences, the loxP sites, is located upstream of a gene of interest, within the same DNA molecule, so as to prevent expression of the later. Expression of the downstream gene occurs only following excision of the loxP-flanked sequences, i.e. after administration of the inducer agent (4-OHT, if the recombinase is Cre-ER^{T}). The originality underlying this approach is that the coding region of an inducible recombinase serves both to control expression of a gene of interest, and is the source of the recombinant activity, preferably inducible, that can permanently either activate or inactivate the expression of said gene of interest. Because of its simplicity and in addition to its in vitro and in vivo experimental potential, this strategy is of interest for the control of therapeutic transgenes, in certain protocols of gene or cell therapy, and in a variety of experimental designs in which conditional expression of genes is required. The system of the invention may be integrated in a DNA vector devised for the transfection of cells.

In a preferred embodiment of the invention, the promoter linked in functional relation to the gene of interest is cell type-specific. Alternatively, said promoter may be inducible. In another embodiment, the polypeptide having a site-specific recombinant activity is Cre; preferably, said polypeptide having a site-specific recombinant activity is Cre-ER^{T}.

Another object of the invention is a DNA construct for the transfection of cells characterized in that it contains at least the system of the invention.

The invention also features a self excision DNA cassette constituted by a DNA sequence flanked by target DNA sequences comprising at least a gene that encodes an inducible polypeptide having a recombinant activity specific for said target DNA sequences. This self-excision DNA cassette may be used as a blocking sequence to temporally prevent the expression of a given gene under the control of a promoter.

A blocking sequence is a DNA sequence of any length whose the presence prevents the expression of a given gene. This can be achieved by either preventing the promoter of the given gene from inducing the transcription of said given gene, or preventing the further translation of the transcribed RNA from the given gene. Thus, the self-excision DNA cassette may be used either as a removable blocking element that prevents the promoter-driven expression of a given gene, until an inducer drug is administered, or as a removable gene which is, on the contrary, constitutively expressed otherwise. A polypeptide having a site-specific recombinant activity is an enzyme that recognizes a particular target sequence and effects the removal of, or otherwise alters the DNA between specific excision sequences.

The advantages of this new system, when compared with previous conditional transgenic systems, are threefold. Both the inductive and effector elements are placed together within a single molecule of DNA, making experimental designs simpler. Moreover, the use of Cre-ER^{T} as site-specific recombinase do not need for permanent administration of the inductive substance, 4-OHT. In addition, it functions independently of the proliferative state of the cells in which the recombinase has been produced, contrary to the HSV-TK/gancyclovir system, in which only dividing cells can be targeted.

This technique should be useful in vivo, in experimental animals or clinical conditions of gene therapy, when irreversible activation of a transgene at a chosen time, bypassing the requirement of constant administration of the inducer molecule, may represent the most suitable approach. Conversely, this technique may be used to irreversibly inactivate the expression of a transgene at will.

### Brief description of the drawings

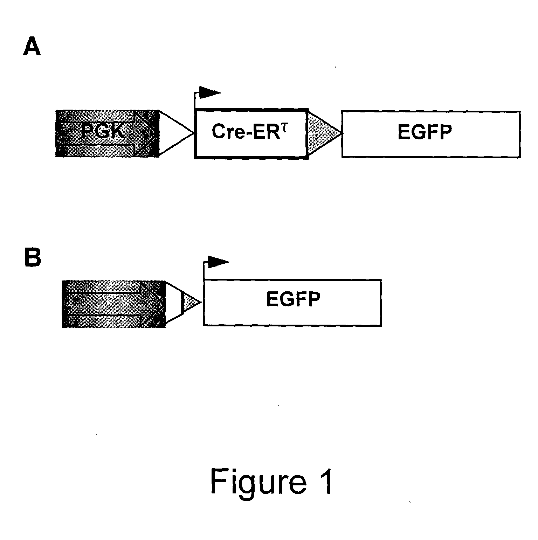

Figure 1 shows transgenes present in the neomycin-resistance gene-containing plasmids used to transfect BHK cells. In figure 1A, the loxP-Cre-ERT-loxP cassette is placed between the mouse phosphoglycerate kinase (PGK) gene promoter and the coding region of the enhanced green fluorescent protein (EGFP). In figure 1B, after excision of the loxP-flanked Cre-ER^{T} gene, the EGFP-coding sequence can be transcribed.

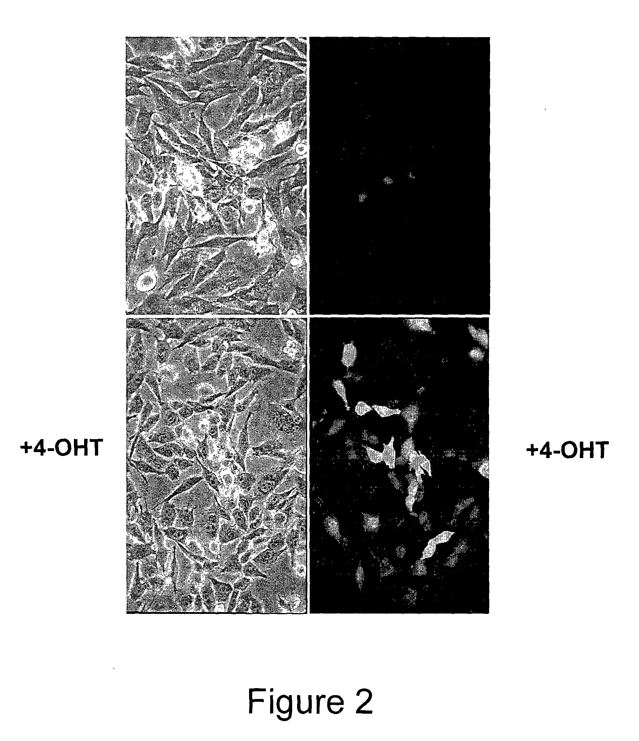

Figure 2 shows dark field photographs of BHK cells transfected with a PGK-loxP-Cre-ER^{T}-loxP-EGFP transgene wherein an expression of EGFP depends on exposure to 4-OHT (down panels). The same fields of cells have been photographed under phase contrast (left panels) or UV (right panels) illumination.

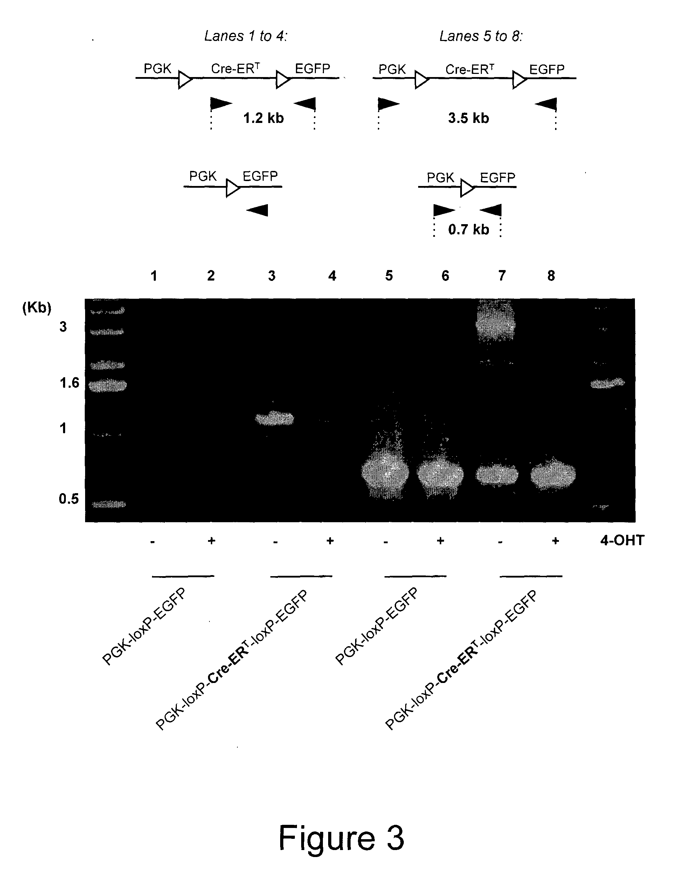

Figure 3 shows a PCR analysis of genomic DNA extracted from transfected BHK cells. Either a PGK-loxP-EGFP (lanes 1, 2, 5 and 6) or a PGK-loxP-Cre-ER^{T}-loxP-EGFP (lanes 3, 4, 7 and 8) transgene was used, and the cells cultured either in the absence (lanes 1, 3, 5,7) or in the presence (lanes 2, 4, 6, 8) of 1 pM 4-OHT. Two types of PCR were performed : i) using 1 oligonucleotide located between the 2 loxP sites (lanes 1 to 4), and ii) using 2 oligonucleotides flanking the loxP-Cre-ER^{T}-loxP cassette (lanes 5 to 8). The 1266 and 3540 bp-long fragments are diagnostic of the non-recombined Cre-ER^{T} containing transgene (lanes 3 and 7, respectively). Using two oligonucleotides flanking the loxP sites, only a fragment of 675 bp is obtained after recombination (lane 8), or from cells bearing the PGK-loxP-EGFP transgene (lanes 5 and 6). In 4-OHT-treated cells bearing the PGK-loxP-Cre-ER^{T}-loxP-EGFP transgene, failure to amplify the 1266 and 3540 bp fragments evidences recombination (lanes 4 and 8). Triangles represent the loxP sequences. Small arrows indicate the relative positions of the oligonucleotides described in the methods section.

Figure 4 shows a design of a "self-excising" bi-cistronic construct, which can be used to achieve the inducible and irreversible repression (excision) of a gene of interest.

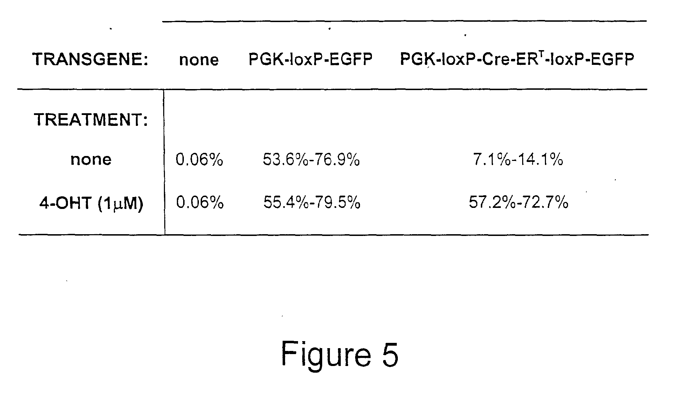

Figure 5 shows a table representing the proportion of fluorescent BHK cells determined by FACS analysis.

### Description of the preferred embodiments

In the following, the gene of interest is a green fluorescent protein (EGFP)-encoding transgene which is linked in functional relation with a phosphoglycerate kinase promoter (PGK). The said second gene that encodes a polypeptide having a recombinant activity specific for target DNA sequences is Cre-ERT, and said two target DNA sequences are loxP.

### Construction of the loxP-flanked Cre-ER^{T} gene

A green fluorescent protein (EGFP)-encoding transgene driven by a phosphoglycerate kinase promoter (PGK) was prepared so as to contain, between the promoter and the coding sequence, a loxP-flanked Cre-ER^{T} gene, as represented in figure 1. A 2 kb EcoRI fragment containing the Cre-ER^{T} gene from plasmid pCMVCre-ER^{T} was cloned into a PBS-KS vector, a Stratagene product, containing a rabbit β-globin polyadenylation (pA) signal as described by Citron, B. et al., *Nucleic Acids Res* 12, 8723-31, 1984. Subsequently, an EcoRI blunted-Kpnl fragment bearing the Cre-ER^{T}/β-globin pA gene was inserted into pBS-246 (Gibco, cat. 10348-019) at Kpnl-EcoRV, between two loxP sites. A Notl blunted-Spel fragment containing the loxP-Cre-ER^{T}-loxP cassette was inserted at sites Xhol blunted-Nhel of pPI-hVEGF165-neo^{r}, immediately downstream of a mouse PGK promoter and a small intron. We termed this plasmid pPl-PGK-loxP-CreER^{T}-loxP-hVEGF-neo^{r}. The HVEGF coding region was then dropped out with a double digestion Notl partial / EcoRI. An EGFP gene-containing Notl-EcoRI fragment from plasmid pEGFP-1 (Clontech, cat. # 6086-1) was used to replace the HVEGF gene, thus yielding the plasmid pPI-PGK-loxP-Cre-ER^{T}-loxP-EGFP-neo^{r} shown in Figure 1A.

Due to basal transcription of the Cre-ER^{T} gene in transformed bacteria, and because the transgene was accessible to the Cre protein in such bacteria, the plasmid DNA preparations obtained contained, together with the full-length non-recombined plasmid (PGK-loxP-Cre-ER^{T}-loxP-EGFP) as shown in Figure 1A, a proportion of plasmid molecules in which the loxP-flanked Cre-ER^{T} sequences had been excised *(PGK-loxP-EGFP*, Figure 1 B).

To transfect BHK cells, given the spontaneous recombination of the Cre-ER T gene in bacterial cells, the plasmid DNA preparations were first purified in 0.7% agarose gels, after linearization with endonuclease Eco47III. This procedure resulted in very low yields of DNA, thus making normal transfection impossible. Thus, the plasmids, containing either the PGK-loxP-Cre-ER^{T}-loxP-EGFP or PGK-loxP-EGFP transgenes (Figure 1) and a neomycin resistance gene, were introduced into BHK cells by direct nuclear microinjection. Therefore, using a phase contrast microscope (Zeiss Axiovert) and a microinjector (Inject+Matic, Geneva), 0.1 to 0.2 picoliters of a solution containing about 50 to 100 DNA molecules were introduced into the nuclei of BHK cells grown to about 50% confluence. About 100 cells were injected per culture dish; a minimum of three dishes was used per construct. G418 (Promega, LT # 90421) (400 pg/ml) was used to select the cells expressing the injected transgene for subsequent experiments.

Cells were maintained in standard culture conditions, at 3.5% CO₂: DMEM 45OmM glucose (GibcoBRL, Cat. # 52100-039) supplemented with 10% FCS (GibcoBRL, Cat. #10106169) and 2mM Glutamine (GibcoBRL, Cat. # 25030-024).

To analyse EGFP expression, emission of fluorescence by either control or transfected BHK cells was monitored using an inverted Zeiss Axiovert microscope (equipped with a U.V. lamp). Cells were also analyzed using a fluorescence-activated cell sorter (model FACScan, Becton Dickinson, USA) after trypsinization.

The loxP-flanked Cre-ER^{T} gene blocks the transcription of a downstream coding sequence (EGFP).

As shown in Figure 5, in the absence of 4-OHT, the loxP-Cre-ER^{T}-loxP cassette efficiently blocked the expression, particularly by blocking the translation, of the EGFP gene: 7% to 14% of the microinjected cells spontaneously expressed EGFP as quantitated by FACS analysis. The presence of a small population of EGFP positive cells presumably correlates with the low level of 4-OHT-independent Cre-ER^{T} recombinant activity reported by others using double transgene systems in vitro.

### Excision of the Cre-ER^{T} gene is regulated by 4-OHT and allows the expression of EGFP.

The 4-OHT treatment began 10 days after the cells had been transfected. 4-OHT (Sigma, Cat. # H-7904) was dissolved in 100% ethanol (BDH, Cat. # 10107) at 1mM; the final concentration was 1 µM (the final concentration of ethanol in the culture dishes was 0.01 %). Cells were analyzed after 4 days of exposure to 4-OHT. In Figure 5, the proportion of EGFP-expressing microinjected cells increased to 57%-73% after exposure to 1 µM 4-OHT. A similar proportion of fluorescent cells was observed in cultures of cells that had been microinjected with the recombined transgene, i.e. PGK-loxP-EGFP, independently of the presence or absence of 4-OHT (54%-80%). These observations were confirmed by PCR analysis of cellular DNA: culture of PGK-loxP-Cre-ER^{T}-loxP-EGFP microinjected BHK cells in the presence of 4-OHT resulted in the near complete removal of the Cre-ER^{T} cassette.

To perform PCR analysis, two types of amplification by PCR were designed to assess for transgene recombination in the DNA extracted from the transfected BHK cells. The first two oligonucleotide primers used were located so that the 5' (sense) oligonucleotide was within the loxP-flanked sequence (ER^{T}s2: 5'-GCT CTA CTT CAT CGC ATT CC-3', on the ER^{T} coding region). The reverse primer, EGFP rev1 (5'-TCG CCC TCG AAC TTC ACC TC-3') was on the EGFP gene. The size of the fragment amplified from the non-recombined transgene is 1266 bp, no amplification being possible after the Cre-mediated excision. The second set of primers were placed flanking the floxed Cre-ER^{T} sequence: PGKpr1 (5'-GTA GCC TTG CAG AAG TTG GTC-3', sense primer, on the PGK promoter region), and EGFPrev1, described above. The intact, non-recombined transgene yields a PCR fragment of 3540 bp, whereas the recombined molecule, after excision of the Cre-ERT sequence, gives a 675-bp band.

## Claims

1. A system to control the expression of a gene of interest comprising
- a first DNA sequence comprising a gene of interest linked in functional relation to a promoter, and
- a second DNA sequence comprising a second gene that encodes a polypeptide having a recombinant activity specific for target DNA sequences, and
- two of said target DNA sequences flanking one of the said two DNA sequences,
**characterized in that** said second DNA sequence is located between the said promoter and said gene of interest.

2. A system of claim 1 **characterized in that** said polypeptide having a recombinant activity is inducible.

3. A system of claim 2 **characterized in that** said second gene is located such that at least said second gene is translated from a single RNA transcript which comprises both the messenger RNA transcribed from the gene of interest and the messenger RNA transcribed from the said second gene.

4. A system of claim 3 **characterized in that** an internal ribosome entry site is placed upstream of said gene of interest.

5. A system of one of the preceding claims **characterized in that** said promoter is tissue-specific or inducible.

6. A system of claim 1 **characterized in that** said polypeptide having a site-specific recombinant activity is Cre.

7. The system of claims 2, 3, 4 or 5 **characterized in that** said polypeptide having a site-specific recombinant activity is Cre-ER^{T}.

8. A DNA vector for the transfection of cells **characterized in that** it contains at least a system of one of the preceding claims.

9. A self excision DNA cassette constituted by a DNA sequence flanked by target DNA sequences comprising at least a gene that encodes a polypeptide having a recombinant activity specific for said target DNA sequences **characterized in that** said polypeptide having a recombinant activity is inducible.

10. The use of the self-excision DNA cassette of claim 9 as a blocking sequence to temporally prevent the expression of a given gene under the control of a promoter.
